# EUROPÄISCHE PATENTSCHRIFT

(11) **EP 1 140 241 B1**
(45) Veröffentlichungstag und Bekanntmachung des Hinweises auf die Patenterteilung: **14.04.2004**
(21) Anmeldenummer: 99967913.7
(22) Anmeldetag: 17.12.1999
(51) Int. Cl.: A61L 27/38

(54) **KARDIOVASKULÄRE PROTHESEN MIT STABILER ENDOTHELZELL-OBERFLÄCHE**
CARDIOVASCULAR PROTHESES WITH A STABLE ENDOTHELIAL CELL SURFACE
PROTHESES CARDIOVASCULAIRES A SURFACE REVETUE D'UNE COUCHE STABLE DE CELLULES ENDOTHELIALES

(30) Priorität: 21.12.1998 DE 19860286
(43) Veröffentlichungstag der Anmeldung: 10.10.2001
(73) Patentinhaber: VasoTissue Technologies GmbH, 10117 Berlin (DE)
(72) Erfinder: PAULITSCHKE, Manrico, D-13086 Berlin (DE); RADEMACHER, Axel, D-10319 Berlin (DE); SITTINGER, Michael, D-15831 Grossziethen (DE)
(74) Vertreter: Wehlan, Helmut, Dr.
(86) Internationale Anmeldenummer: PCT/DE1999/004102
(87) Internationale Veröffentlichungsnummer: WO 2000/037123

(56) Entgegenhaltungen:
- EP-A- 0 320 441
- WO-A-93/01843
- WO-A-97/49799

## Beschreibung

Die Erfindung betrifft kardiovaskuläre Prothesen mit stabiler, durch Proliferation unter Scherstress erzeugter konfluenter Endothelzell-Oberfläche, die durch ein neuartiges Verfahren zur Ausbildung eines stabilen konfluenten Endothelzellmonolayers geschaffen werden. Unter dem Begriff kardiovaskuläre Prothesen werden dabei Gefäß- und Herzklappenprothesen verstanden, die blutkontaktseitig mit Endothelzellen beschichtet werden.

Verfahren zur blutkontaktseitigen Auskleidung von kardiovaskulären Prothesen mit Endothelzellen sind bekannt, auch solche, bei denen das Erreichen eines konfluenten Endothelzellmonolayers durch Wachstum der Zellen unter statischen Bedingungen nach initialer subkonfluenter Besiedlung oder durch eine direkte konfluente Besiedlung der isolierten Zellen (US-Patent 5334879) erfolgt. Dabei kann die Besiedlung unter schrittweiser und/oder permanenter Rotation wie auch statisch vorgenommen werden.

Ein Verfahren zur Herstellung von vaskulären Transplantaten wird in der Druckschrift WO 97/49799 A beschrieben. Dabei steht das Transplantat in Kontakt mit einem flüssigen Medium und ist während der Besiedlung physiologischen Bedingungen entsprechenden Scherkräften ausgesetzt. Die Scherkräfte werden dabei durch das Pumpen einer Flüssigkeit direkt durch das Transplantat erzeugt. Die Flüssigkeit kann entweder Zellen oder Kulturmedium enthalten. Die Zellen werden mittels Durchströmung eingebracht.

Implantate aus Zellkulturen können auch mittels einer resorbierbaren Trägerstruktur nach Ummantelung der Zellen (DE 43 06 661) sowohl als 3-dimensionales als auch als 2-dimensionales Implantat hergestellt werden, wobei verschieden erzeugte Trägermaterialien möglich sind (WO 94/7 7841). In der Schrift EP 320 441 wird ein Verfahren zum Konditionieren von mit lebenden Zellen beschichteten Kunststoffträgern beschrieben - die Anpassung an einen Scherstress soll erreicht werden. Dabei wird ein zeitlich unterbrochener Medienstrom verwendet - ein permanenter Scherstress wirkt nicht ein. Es handelt sich hierbei um die Konditionierung des mit Zellen konfluent belegten Trägers und eine Gewöhnung der abgelagerten Zellen an den Scherstress, nicht um eine Adaption der Zellen bereits während ihres Teilungs- und Anheftungsprozesses.

Der Eintrag von Scherkräften ist auch Gegenstand der Schrift WO 93/01843. Es werden hiernach jedoch nur Scherkräfte sehr geringer Größe, vor allem aber radialer Orientierung, eingetragen. Dabei erfolgt das Ansiedeln der Zellen unter statischen Bedingungen, bei einer 4-fachen Rotation der Prothese alle 20 min um jeweils 90°C, und erst danach wird ein permanenter Scherstress sehr geringer Größe durch Rotation auf einem Roller (10 U/min) erzeugt.

Über die Bildung eines geschlossenen Zellmonolayers berichtet die Offenlegungsschrift WO 94/25584. Dieser wird sofort bei der Ansiedlung erzeugt. Die initiale Adhäsion findet unter statischen Bedingungen statt. Danach erfolgt über mehr als 24 h die Inkubation des konfluent ausgesäten Monolayers, auch hier erfolgt die Anpassung der Zellen an physiologische Größen von Scherstress nicht während der Proliferation der Zellen. Im US-Patent 5634879 wird über ein Verfahren berichtet, das eine direkte Aussaat der Zellen auf die Prothese nach der Isolation vorsieht, ohne das Ziel zu verfolgen, eine Anpassung der Zellen an einen erhöhten definierten Scherstress zu erhalten. Dabei werden die Zellen durch eine transversale Filtration der Suspension durch das Protheserunaterial hindurch auf die innere Oberfläche aufgebracht. Eine geschlossene Kreislaufperfusion liegt nicht vor. Es erfolgt auch keine Proliferation unter definierten Strömungsbedingungen.

Der Nachteil der beschriebenen Erfindungen besteht darin, dass entweder das Proliferationsverhalten der Zellen unter statischen Bedingungen, bedingt durch eine veränderte Genexpression, eine verminderte Haftung der Zellen auf der Prothesenoberfläche zur Folge hat oder in vitro keine Bedingungen simuliert werden, die denen der in vivo Situation entsprechen.

Die Aufgabe der Erfindung besteht darin, kardiovaskuläre Prothesen zu entwickeln, mit denen sich den genannten Nachteilen begegnen lässt.

Die Aufgabe wurde dadurch gelöst, dass in kardiovaskulären Prothesen mit Endothelzell-Oberfläche nach einer initial subkonfluenten Besiedlung der blutkontaktseitigen Oberfläche die Ausbildung eines konfluenten Monolayers erfolgt. Das geschieht durch Wachstum der Zellen unter permanenter Einwirkung von bis auf physiologische Werte ansteigenden definierten pulsatilen Scherkräften mittels Umströmung der blutkontaktseitigen Prothesenoberfläche längs der Prothesenhauptachse in einem inneren Perfusionskreislauf und einer Benetzung der Prothesenaußenwand in einem äußeren Perfusionskreislauf bzw. durchströmbaren Medienreservoir.

Das Wesen der Erfindung liegt in einer Kombination aus bekannten (Verfahren zur Ausbildung eines konfluenten Monolayers auf der Oberfläche von kardiovaskulären Prothesen) und neuen Elementen (Anpassung der Endothelzellen an einen im Blutgefäßsystem lokal am Implantationsort vorhandenen hämodynamischen Scherstress), die sich gegenseitig beeinflussen und in ihrer neuen Gesamtwirkung einen Gebrauchsvorteil und den erstrebten Erfolg ergeben, der darin liegt, dass der hämodynamische Scherstress direkten Einfluss auf Struktur und Funktion der Endothelzellen nimmt und damit bereits während ihres Wachstums (Zellteilungsphase) Einfluss auf die Bildung eines konfluenten Endothelzellmonolayers bewirkt.

Überraschenderweise hat sich herausgestellt, dass während der Ausbildung des konfluenten Endothelzellmonolayers durch Zellteilung die Zellpopulation bereits an Wandschubspannungen, wie sie in vivo zu beobachten sind, adaptiert wird, mit der Folge einer stabilen Haftung der Zellen auf der blutkontaktseitigen Prothesenoberfläche, welche für die langfristige strukturelle und funktionelle Wirksamkeit des Endothelzellmonolayers als Grenzschicht zwischen Prothesenoberfläche und dem strömenden Blut von entscheidender Bedeutung ist. Damit wird der Aufbau einer neuartigen Prothesenoberfläche erreicht, die vergleichbar mit der in vivo Auskleidung von Blutgefäßen ist und daraus folgend eine deutliche Reduzierung des Gerinnungsrisikos im Vergleich zu unbeschichteten oder nicht konfluent mit Endothelzellen ausgekleideten Prothesen erreicht wird. Bereits bekannte Verfahren sind durch eine initiale und unter statischen Bedingungen erfolgende Besiedlung der Prothesenoberfläche gekennzeichnet, die nur kurzfristig und diskontinuierlich zum Austausch des Mediums unterbrochen wird (EP 0 320 441). Bekannt sind des weiteren Verfahren, in denen die Einstellung der Adhärenz der Zellen auf der Prothesenoberfläche immer mit der Ausbildung der unmittelbaren Konfluenz (WO 93/01843) einhergeht. Es erfolgt daher keine Proliferation der Endothelzellen unter permanentem Einwirken von definierten pulsatilen Scherkräften (WO 94/2584).

Die Erfindung gestattet u.a. eine Adaption der Zellen an verschiedene Wandschubspannungen unter Berücksichtigung der später im Implantat lokal auftretenden Scherkräfte in Abhängigkeit vom Implantationsort bereits vor der Implantation der Prothese in den Blutkreislauf.

Die ansteigenden Scherkräfte können mittels einer programmgesteuerten Pumpeinrichtung (7) erzeugt werden. Die mathematische Größe der ansteigenden Scherkräfte kann variabel und zeitlich unabhängig gewählt werden. Gemäß einer vorteilhaften Ausgestaltung lassen sich die mathematische Größe und der Endwert der Scherkräfte adäquat den physiologischen Bedingungen des Implantationsorts frei und zeitvariabel über eine Programmsteuerung wählen und ermöglichen damit die Ausbildung sowohl arterieller als auch venöser Gefäßprothesen (1) entsprechend der unterschiedlichen, in vivo auftretenden Wandschubspannungen und die Anpassung an pulsatile Strömungsbedingungen.

Gemäß einer weiteren vorteilhaften Ausgestaltung kann die mathematische Größe der auftretenden Scherkräfte sowohl durch Variation der Pumpleistung als auch durch Variation der Größe des Querschnittes der verwendeten Pumpschläuche oder anderer Verbindungselemente außerhalb der Kammer sowie durch die geometrische Form und Ausführung der Kammer selbst eingestellt werden.

Der äußere Perfusionskreislauf (5') kann gemäß einer weiteren vorteilhaften Ausgestaltung im Gleich- oder Gegenstrom zum inneren Perfusionskreislauf (5), aber auch statisch betrieben werden. Beide Perfusionskreisläufe (5,5') können auch als nicht geschlossenes System arbeiten; sie führen gemäß einer weiteren vorteilhaften Ausgestaltung von einem Medienreservoir (6) in ein anderes Medienreservoir (6'), in dem das Medium, das die Prothese bereits durchströmt hat, aufgefangen wird. Dabei können beide Kreisläufe auch innerhalb der Kammer (2) nach Umströmung der Prothesenoberfläche zusammengeführt werden.

Innerer und äußerer Perfusionskreislauf können verschiedene Reservoire oder ein und dasselbe Medienreservoir (6, 6') besitzen. Die Prothese kann sich im Medienreservoir selbst befinden, und dadurch können innerer und äußerer Perfusionskreislauf miteinander verbunden werden.

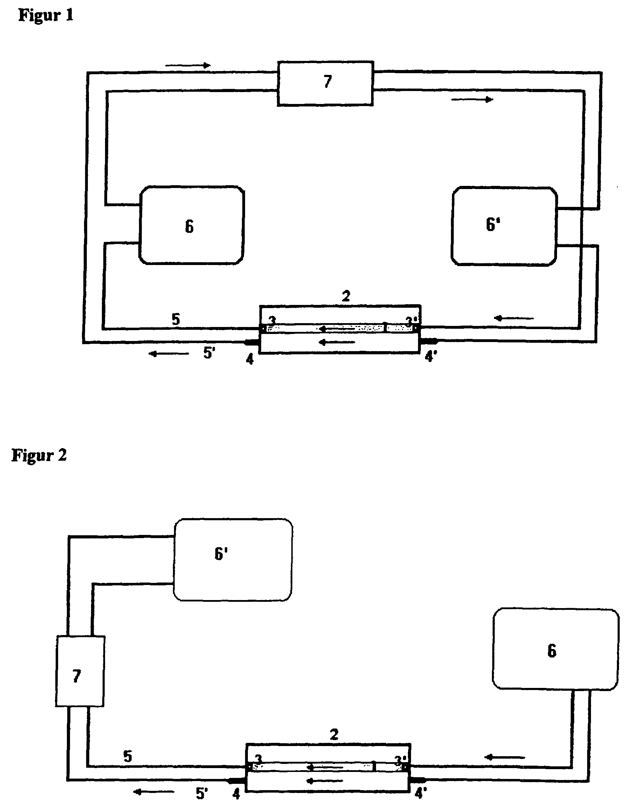

Die sich an die Besiedlung der blutkontaktseitigen Prothesenoberfläche anschließende Ausbildung eines konfluenten Endothelzellmonolayers erfolgt mittels eines Perfusionskreislaufs, dargestellt in Figur 1 und Figur 2. Er besteht aus einem inneren Perfusionskreislauf zur Umströmung der blutkontaktseitigen Prothesenoberfläche längs der Prothesenhauptachse im Inneren einer Perfusionskammer, wobei die Prothese mittels Adapter im Innenraum der Perfusionskammer befestigt und ausgerichtet wird und somit selbst einen Teil des inneren Perfusionskreislaufs bildet sowie aus einem äußeren Perfusionskreislauf zur äußeren Umströmung der Gefäßprothese in derselben Perfusionskammer, welche nach außen hin für beide Kreisläufe Verbindungen zu einer Pumpeinrichtung sowie zu den steril auswechselbaren Medienreservoiren, welche auch als Druckausgleichsbehälter fungieren, aufweisen.

Der äußere Perfusionskreislaufs dient der Benetzung der Prothesenaußenseite, um ihr Austrocknen zu verhindern. Das Prothesenmaterial ist häufig von hoher Porosität und kann vor der Zellaussaat mit Fibrin oder anderen adhäsionsfördernden Substanzen durchtränkt werden. Eine optionale Perfusion verhindert den Aufbau möglicher Gradienten sowohl in der Medienzusammensetzung als auch des pH-Wertes an der und/oder durch die Prothesenwandung. Damit wird eine gradientenabhängige transversale Diffusion durch das Prothesenmaterial hindurch verhindert.

Die Erfindung bezieht sich mithin auf durch ein neuartiges Verfahren erzeugte kardiovaskuläre Prothesen, gekennzeichnet durch die Ausbildung eines stabilen konfluenten Endothelzellmonolayers auf der blutkontaktseitigen Oberfläche der Prothesen. Dabei erfolgt sowohl die initiale Besiedlung der blutkontaktseitigen Prothesenoberfläche als auch das sich anschließende Wachstum der Endothelzellen zu einem konfluenten Monolayer unter dem permanenten Einfluss von Scherstress, welcher in zwei Etappen erzeugt wird: anfangs in der Besiedlungsphase zur Erzeugung eines subkonfluenten Endothelzellmonolayer durch die axiale Rotation der Kulturkammer sowie anschließend durch die Umströmung der Prothesenoberfläche längs der Prothesenhauptachse. Damit werden in vitro auf der blutkontaktseitigen Prothesenoberfläche Bedingungen geschaffen, die vergleichbar mit der in vivo Situation sind. Daraus folgend wird eine konfluente Endothelzellschicht mit hoher Qualität gebildet.

Diese neuartigen kardiovaskulären Prothesen erzielen eine deutlich verbesserte Haftung der Zellen auf der blutkontaktseitigen Prothesenoberfläche und ermöglichen somit den Erhalt des Monolayers auch langfristig und unter erhöhten Scherstressbedingungen. Damit wird erstmalig eine deutliche Reduzierung des Gerinnungsrisikos im Vergleich zu unbeschichteten, nicht konfluent mit Endothelzellen ausgekleideten Prothesen wie auch zu Prothesen, die konfluent besiedelt wurden, aber eine unzureichende Haftung der Zellen auf der blutkontaktseitigen Prothesenoberfläche aufweisen, erreicht. Dieses Verfahren eignet sich für die Beschichtung sowohl von Herzklappen und Gefäßprothesen, wie sie in der kardiovaskulären Chirurgie zur Anwendung kommen, als auch von Stents.

Das erfindungsgemäße Verfahren besteht darin, dass nach einer initial subkonfluenten Besiedlung der blutkontaktseitigen Prothesenoberfläche die Ausbildung eines konfluenten Monolayers durch Wachstum der Zellen unter permanenter Einwirkung-von bis auf physiologische Werte ansteigenden definierten pulsatilen Scherkräften mittels Umströmung der Prothesenoberfläche längs der Prothesenhauptachse in einem inneren Perfusionskreislauf und einer Benetzung der Prothesenaußenwand in einem äußeren Perfusionskreislauf oder in einem durchströmbaren Medienreservoir erfolgt.

Weitere Ausgestaltungen werden in den Ansprüchen beschrieben.

Die Erfindung soll anhand von Ausführungsbeispielen näher erläutert werden, ohne auf diese Beispiele beschränkt zu sein.

### Ausführungsbeispiele

### Beispiel 1:

Eine Polytetrafluorethylen-Bypass-Prothese (Fig. 1: 1) mit einem Durchmesser von 4 mm und einer Länge von 15 cm wird mit Hilfe der Adapter (Fig. 1: 3,3'), die als Olive, Kegel mit Spannvorrichtung oder Spreizvorrichtung ausgebildet sind, am Innenraum der Perfusionskammer (Fig. 1: 2) befestigt und somit mit dem inneren Perfusionskreislauf (Fig. 1: 5) verbunden. Die pulsatile Strömung (0 - 3.500 ml/h Endothelzellkulturmedium) wird über eine Peristaltikpumpe, die von Hand oder programmgesteuert frei und zeitvariabel geregelt werden kann (Fig. 1: 7), erzeugt. Über die Schlauchanschlüsse (Fig. 1: 4,4'), die dezentral an den Seiten der Perfusionskammer angebracht sind, wird mit Silikonschläuchen der äußere Perfusionskreislauf (Fig. 1: 5') hergestellt, der im Gleichstrom zum inneren Kreislauf perfundiert wird. Die beiden Perfusionskreisläufe (Fig. 1: 5,5') verfügen jeweils über ein Reservoir (Fig. 1: 6,6'), das Endothelzellkulturmedium enthält und gleichzeitig als Druckausgleichsbehälter fungiert. Die Peristaltikpumpe (Fig. 1: 7) wird über Computer so gesteuert, dass die über eine pulsatile Strömung eingetragenen Scherkräfte in der Prothese über einen Zeitraum von 10 h von 0,001 auf 0,25 N/m² (0,01 auf 2,5 dyn/cm²) ansteigen. Danach bleiben die Strömungsbedingungen bis zum Tag der Implantation konstant.

Anschließend wird die Kammer in steriler Umgebung geöffnet, und die konfluent mit Zellen beschichtete Prothese kann implantiert werden.

### Beispiel 2:

Eine Dacron®-Bypass-Prothese (Fig. 1: 1) mit einem Durchmesser von 5 mm und einer Länge von 10 cm wird mit Hilfe der Adapter (Fig. 1: 3,3') am Innenraum der Perfusionskammer (Fig. 1: 2) befestigt und somit mit dem inneren Perfusionskreislauf (Fig. 1: 5) verbunden. Die pulsatile Strömung (0 - 7.000 ml/h Endothelzellkulturmedium) wird über eine Peristaltikpumpe, die von Hand oder programmgesteuert frei und zeitvariabel geregelt werden kann (Fig. 1: 7), erzeugt. Der äußere Perfusionskreislauf wird (Fig. 1: 5') mit Medium gefüllt und statisch betrieben. Die beiden Perfusionskreisläufe (Fig. 1: 5,5') verfügen über ein gemeinsames Reservoir, das Endothelzellkulturmedium enthält und gleichzeitig als Druckausgleichsbehälter dient. Verwendung finden hierbei expandierbare Blutbeutel der Materialien Ethylenvinylacetat-M (EVAM) oder Polyvinylchlorid (PVC).

Die Peristaltikpumpe (Fig. 1: 7) wird über Computer so gesteuert, dass die über eine pulsatile Strömung eingetragenen Scherkräfte in der Prothese über einen Zeitraum von 24 h von 0,001 auf 0,5 N/m² (0,01 auf 5 dyn/cm²) ansteigen. Danach bleiben die Strömungsbedingungen bis zum Tag der Implantation konstant.

Anschließend wird die Kammer in steriler Umgebung geöffnet, und die konfluent mit Zellen beschichtete Prothese kann implantiert werden.

### Beispiel 3:

Eine Polytetrafluorethylen-Gefäßprothese (Fig. 1: 1) mit einem Durchmesser von 10 mm und einer Länge von 12 cm wird mit Hilfe eines Adapters im Innenraum des Medienreservoirs, das somit den äußeren Perfusionskreislauf darstellt, befestigt. Die pulsatile Strömung (0 - 7.000 ml/h Endothelzellkulturmedium) wird über eine Peristaltikpumpe, die von Hand oder programmgesteuert frei und zeitvariabel geregelt werden kann (Fig. 1: 7), erzeugt, wobei der Innendurchmesser der Pumpschläuche variabel ist. Die Peristaltikpumpe (Fig. 1: 7) wird über Computer so gesteuert, dass die über eine pulsatile Strömung eingetragenen Scherkräfte in der Prothese über einen Zeitraum von 24 h von 0,001 auf 0,5 N/m² (0,01 auf 5 dyn/cm²) ansteigen. Danach bleiben die Strömungsbedingungen bis zum Tag der Implantation konstant.

Anschließend wird die Kammer in steriler Umgebung geöffnet, und die konfluent mit Zellen beschichtete Prothese kann implantiert werden.

### Beispiel 4:

Eine Polytetrafluorethylen-Bypass-Prothese (Fig. 1: 1) mit einem Durchmesser von 4 mm und einer Länge von 4 cm wird mit Hilfe der Adapter (Fig. 1: 3,3'), die als Olive, Kegel mit Spannvorrichtung oder Spreizvorrichtung ausgebildet sind, am Innenraum der Perfusionskammer (Fig. 1: 2) befestigt und somit mit dem inneren Perfusionskreislauf (Fig. 1: 5) verbunden. Die pulsatile Strömung (0 - 3.500 ml/h Endothelzellkulturmedium) wird über eine Peristaltikpumpe, die von Hand oder programmgesteuert frei und zeitvariabel geregelt werden kann (Fig. 1: 7), erzeugt. Über die Schlauchanschlüsse (Fig. 1: 4,4'), die dezentral an den Seiten der Perfusionskammer angebracht sind, wird mit Silikonschläuchen der äußere Perfusionskreislauf (Fig. 1: 5') hergestellt, der im Gegenstrom zum inneren Kreislauf perfundiert wird. Hierbei erfolgt die Perfusion eines oder beider Kreisläufe in einem nicht geschlossenen System. Dabei strömt Medium von einem als Vorrat dienenden Medienreservoir in ein anderes, als Auffangbehälter dienendes Reservoir.

Die Peristaltikpumpe (Fig. 1: 7) wird über Computer so gesteuert, dass die über eine pulsatile Strömung eingetragenen Scherkräfte in der Prothese über einen Zeitraum von 10 h von 0,001 auf 0,25 N/m² (0,01 auf 2,5 dyn/cm²) ansteigen. Danach bleiben die Strömungsbedingungen bis zum Tag der Implantation konstant.

Anschließend wird die Kammer in steriler Umgebung geöffnet, und die konfluent mit Zellen beschichtete Prothese kann implantiert werden.

### Beispiel 5:

Eine Herzklappenprothese (Fig. 1: 1) wird mit Hilfe eines Adapters (Fig. 1: 3) am Innenraum der Perfusionskammer (Fig. 1: 2) befestigt und somit mit dem inneren Perfusionskreislauf (Fig. 1: 5) verbunden. Die pulsatile Strömung (0 - 7.000 ml/h Endothelzellkulturmedium) wird über eine Peristaltikpumpe, die von Hand oder programmgesteuert frei und zeitvariabel geregelt werden kann (Fig. 1: 7), erzeugt. Der äußere Perfusionskreislauf wird (Fig. 1: 5') im Gleichstrom perfundiert. Nach Umströmung der Herzklappenprothese vereinigen sich beide Perfusionskreisläufe im Inneren der Kammer (2) bis zum Verlassen derselben. Die beiden Perfusionskreisläufe (Fig. 1: 5,5') verfügen über ein gemeinsames Reservoir, das Endothelzellkulturmedium enthält und gleichzeitig als Druckausgleichsbehälter dient. Verwendung finden hierbei expandierbare Blutbeutel der Materialien Ethylenvinylacetat-M (EVAM) oder Polyvinylchlorid (PVC).

Die Peristaltikpumpe (Fig. 1: 7) wird über Computer so gesteuert, dass die über eine pulsatile Strömung eingetragenen Scherkräfte in der Prothese über einen Zeitraum von 24 h von 0,001 auf 0,5 N/m² (0,01 auf 5 dyn/cm²) ansteigen. Danach bleiben die Strömungsbedingungen bis zum Tag der Implantation konstant.

Anschließend wird die Kammer (2) in steriler Umgebung geöffnet, und die konfluent mit Zellen beschichtete Prothese kann implantiert werden.

### Figuren

Figur 1 zeigt den schematischen Aufbau eines geschlossenen Perfusionssystems. Hauptelement bildet die Kammer (2), die über die Schlauchanschlüsse (4,4') zum inneren Kreislauf (5) als auch zum äußeren Kreislauf (5') mit der Pumpeinrichtung (7) sowie den zugehörigen Medienreservoiren (6,6') verbunden ist. Der innere Kreislauf wird durch die über die Adapter (3,3') in der Kammer fixierte Gefäßprothese (1) geschlossen. Die dargestellten Pfeile symbolisieren die Strömungsrichtung des perfundierenden Mediums, wobei in dieser Darstellung eine Perfusion beider Kreisläufe in Gleichstrom erfolgt.

Figur 2 zeigt den schematischen Aufbau eines Perfusionssystems gemäß Figur 1, jedoch erfolgt hier die Perfusion nicht mittels zweier geschlossener Kreisläufe, sondern von einem Medienreservoir (6) in ein anderes Medienreservoir (6'), in dem das Medium, das die Prothese bereits durchströmt hat, aufgefangen wird.

### Bezugszeichen

- 1 -: kardiovaskuläre Prothese
- 2 -: Kammer
- 3,3' -: Adapter
- 4,4' -: Schlauchanschluss
- 5 -: innerer Kreislauf
- 5' -: äußerer Kreislauf
- 6,6' -: Medienreservoir
- 7 -: Pumpe

## Patentansprüche

1. Kardiovaskuläre Prothesen mit Endothelzell-Oberfläche, herstellbar dadurch, dass nach einer initial subkonfluenten Besiedlung der blutkontaktseitigen Oberfläche die Ausbildung eines konfluenten Monolayers durch Wachstum der Zellen unter permanenter Einwirkung von bis auf physiologische Werte ansteigenden definierten pulsatilen Scherkräften mittels Umströmung der blutkontaktseitigen Prothesenoberfläche längs der Prothesenhauptachse in einem inneren Perfusionskreislauf und einer Benetzung der Prothesenaußenwand in einem äußeren Perfusionskreislauf oder in einem durchströmbaren Medienreservoir erfolgt.

2. Kardiovaskuläre Prothesen nach Anspruch 1, **dadurch gekennzeichnet, dass** die ansteigenden Scherkräfte mittels einer programmgesteuerten Pumpeinrichtung (7) erzeugt werden.

3. Kardiovaskuläre Prothesen nach Anspruch 1 und 2, **dadurch gekennzeichnet, dass** die mathematische Größe der ansteigenden Scherkräfte variabel und zeitlich unabhängig gewählt werden kann.

4. Kardiovaskuläre Prothesen nach Anspruch 1 bis 3, **dadurch gekennzeichnet, dass** die mathematische Größe und der Endwert der Scherkräfte adäquat den physiologischen Bedingungen des Implantationsorts frei und zeitvariabel über eine Programmsteuerung gewählt werden können.

5. Kardiovaskuläre Prothesen nach Anspruch 1 bis 4, **dadurch gekennzeichnet, dass** die mathematische Größe der auftretenden Scherkräfte sowohl durch Variation der Pumpleistung als auch durch Variation der Größe des Querschnittes der verwendeten Pumpschläuche oder anderer Verbindungselemente außerhalb der Kammer sowie durch die geometrische Form und Ausführung der Kammer selbst eingestellt werden kann.

6. Kardiovaskuläre Prothesen nach Anspruch 1 bis 5, herstellbar mittels eines Perfusionskreislaufs, bestehend aus einem inneren Perfusionskreislauf (5) zur Umströmung der blutkontaktseitigen Prothesenoberfläche längs der Prothesenhauptachse im Inneren der Kammer (2), wobei die Prothese (1) mittels Adapter (3,3') im Innenraum befestigt wird und somit selbst den inneren Perfusionskreislauf (5) bildet sowie einem äußeren Perfusionskreislauf (5') zur äußeren Umströmung der Prothese (1) in derselben Kammer (2), welche nach außen hin für beide Kreisläufe (5,5') Verbindungen zu einer Pumpeinrichtung (7) sowie den Medienreservoiren (6,6'), welche auch als Druckausgleichsbehälter fungieren, aufweist.

7. Kardiovaskuläre Prothesen nach Anspruch 1 bis 6, herstellbar mittels eines Perfusionskreislaufs, bestehend aus einem inneren Perfusionskreislauf (5) zur Umströmung der blutkontaktseitigen Prothesenoberfläche längs der Prothesenhauptachse im Inneren der Kammer (2), wobei die Prothese (1) mittels eines Adapters (3,) im Innenraum befestigt wird und somit selbst den inneren Perfusionskreislauf (5) bildet sowie einem äußeren, sich mit dem inneren Perfusionskreislauf (5) nach Umströmen der Prothese (1) im Inneren der Kammer (2) vereinigenden Perfusionskreislauf (5') zur äußeren Umströmung der Prothese (1) in derselben Kammer (2), welche nach außen hin für beide Kreisläufe (5,5') Verbindungen zu einer Pumpeinrichtung (7) sowie den Medienreservoiren (6,6'), welche auch als Druckausgleichsbehälter fungieren, aufweist.

8. Kardiovaskuläre Prothesen nach Anspruch 6 und 7, **dadurch gekennzeichnet, dass** der äußere Perfusionskreislauf (5') im Gleich- oder Gegenstrom zum inneren Perfusionskreislauf (5), aber auch statisch betrieben werden kann.

9. Kardiovaskuläre Prothesen nach Anspruch 6 bis 8, **dadurch gekennzeichnet, dass** die Perfusionskreisläufe von einem Medienreservoir (6) in ein anderes Medienreservoir (6') führen, in dem das Medium, das die Prothese bereits durchströmt hat, aufgefangen wird.

10. Kardiovaskuläre Prothesen nach Anspruch 6 bis 8, **dadurch gekennzeichnet, dass** innerer und äußerer Perfusionskreislauf verschiedene Medienreservoire oder ein und dasselbe Medienreservoir (6,6') besitzen.

11. Kardiovaskuläre Prothesen nach Anspruch 6 bis 8, **dadurch gekennzeichnet, dass** die Prothese sich im Medienreservoir selbst befindet und dadurch innerer und äußerer Perfusionskreislauf miteinander verbunden sind.

12. Kardiovaskuläre Prothesen nach Anspruch 6 bis 11, **dadurch gekennzeichnet, dass** die Medienreservoire aus expandierbaren Blutbeuteln der Materialien PVC oder EVAM bestehen.

13. Kardiovaskuläre Prothesen nach Anspruch 6 und 7, **dadurch gekennzeichnet, dass** die Ausführung der Adapter (3,3') zur Befestigung der Prothese (1) als Olive, Kegel mit Spannvorrichtung oder als Spreizelement möglich ist.

14. Kardiovaskuläre Prothesen nach Anspruch 6, 7 und 13, **dadurch gekennzeichnet, dass** die Länge der einzuspannenden Prothese durch die konstruktive Auslegung mindestens eines Abschlussteils mit dem Adapter (3 oder 3') der Kammer (1) variiert werden kann.

15. Kardiovaskuläre Prothesen nach Anspruch 6 und 7, **dadurch gekennzeichnet, dass** die Kammer (2) aus einem durchsichtigen Material gefertigt wird.

16. Kardiovaskuläre Prothesen nach Anspruch 1 bis 15, **dadurch gekennzeichnet, dass** als Prothese eine Gefäßprothese, eine Herzklappenprothese oder ein Stent verwendet wird.

17. Verfahren zur Beschichtung von kardiovaskulären Prothesen mit Endothelzellen nach Anspruch 1 bis 16, **dadurch gekennzeichnet, dass** nach einer initial subkonfluenten Besiedlung der blutkontaktseitigen Prothesenoberfläche die Ausbildung eines konfluenten Monolayers durch Wachstum der Zellen unter permanenter Einwirkung von bis auf physiologische Werte ansteigenden definierten pulsatilen Scherkräften mittels Umströmung der blutkontaktseitigen Prothesenoberfläche längs der Prothesenhauptachse in einem inneren Perfusionskreislauf und einer Benetzung der Prothesenaußenwand in einem äußeren Perfusionskreislauf oder in einem durchströmbaren Medienreservoir erfolgt.

18. Verfahren nach Anspruch 17, **dadurch gekennzeichnet, dass**
a) die ansteigenden Scherkräfte mittels einer programmgesteuerten Pumpeinrichtung (7) erzeugt werden.
b) die mathematische Größe der ansteigenden Scherkräfte variabel und zeitlich unabhängig gewählt werden kann
c) die mathematische Größe und der Endwert der Scherkräfte adäquat den physiologischen Bedingungen des Implantationsorts frei und zeitvariabel über eine Programmsteuerung gewählt werden können
d) die mathematische Größe der auftretenden Scherkräfte sowohl durch Variation der Pumpleistung als auch durch Variation der Größe des Querschnittes der verwendeten Pumpschläuche oder anderer Verbindungselemente außerhalb der Kammer sowie durch die geometrische Form und Ausführung der Kammer selbst eingestellt werden kann.

19. Verfahren nach Anspruch 17 und 18, **dadurch gekennzeichnet, dass** in einem inneren Perfusionskreislauf (5) zur Durchströmung des Protheseninnenraums längs der Prothesenhauptachse im Inneren der Kammer (2) die Prothese (1) mittels Adapter (3,3') im Innenraum befestigt wird und somit selbst den inneren Perfusionskreislauf (5) bildet und dass ein äußerer Perfusionskreislauf (5') zur äußeren Umströmung der Prothese (1) in derselben Kammer (2), welche nach außen hin für beide Kreisläufe (5,5') Verbindungen zu einer Pumpeinrichtung (7) sowie den Medienreservoiren (6,6'), welche auch als Druckausgleichsbehälter fungieren, vorhanden ist.

20. Verfahren nach Anspruch 17 bis 19, **dadurch gekennzeichnet, dass**
a) der äußere Perfusionskreislauf (5') im Gleich- oder Gegenstrom zum inneren Perfusionskreislauf (5), aber auch statisch betrieben werden kann
b) beide Perfusionskreisläufe (5,5') nicht als geschlossenes System arbeiten, sondern von einem Medienreservoir (6) in ein anderes Medienreservoir (6') führen, in dem das Medium, das die Prothese bereits durchströmt hat, aufgefangen wird
c) innerer und äußerer Perfusionskreislauf verschiedene Medienreservoire oder ein und dasselbe Medienreservoir (6,6') besitzen
d) beide Perfusionskreisläufe (5,5') sich nach Umströmen der Prothese (1) in Inneren der Kammer (2) vereinigen, aber in getrennten Perfusionskreisläufen (5,5') die Kammer (2) verlassen.

21. Verfahren nach Anspruch 17 bis 20, **dadurch gekennzeichnet, dass** die Prothese sich im Medienreservoir selbst befindet und dadurch innerer und äußerer Perfusionskreislauf miteinander verbunden sind.

## Claims

1. Cardiovascular prostheses with an endothelial cell surface, which can be produced in that, after an initial subconfluent population of the blood contact side prosthesis surface, the formation of a confluent monolayer takes place by cell growth under permanent influence of defined pulsatile shearing forces increasing up to physiological values, by exposing the blood contact side prosthesis surface to a flow along the prosthesis main axis in an inner perfusion circuit, and by wetting the outer prosthesis wall in an outer perfusion circuit or in a fluid reservoir that may be flown through.

2. Cardiovascular prostheses according to claim 1, **characterized in that** the increasing shearing forces are generated by means of a program-controlled pumping means (7).

3. Cardiovascular prostheses according to claims 1 and 2, **characterized in that** the mathematical quantity of the increasing shearing forces may be selected variably and independent of time.

4. Cardiovascular prostheses according to claims 1 through 3, **characterized in that** the mathematical quantity and the final value of the shearing forces may be selected freely and time-variably in adequacy to the physiological conditions of the implantation site via a program control.

5. Cardiovascular prostheses according to claims 1 through 4, **characterized in that** the mathematical quantity of the shearing forces occurring may be adjusted both by variation of the pumping performance and by variation of the cross-sectional size of the pumping tubes used or of other connection members outside of the chamber, as well as by the geometrical shape and configuration of the chamber itself.

6. Cardiovascular prostheses according to claims 1 through 5, which can be produced by means of a perfusion circuit comprised of an inner perfusion circuit (5) for exposing the blood contact side prosthesis surface to a flow along the main prosthesis axis within the chamber (2), with the prosthesis (1) being attached in the inner space by means of adapters (3, 3'), and the prosthesis itself thus forming the inner perfusion circuit (5), as well as an outer perfusion circuit (5') for exposing the prosthesis (1) to an outer flow within the same chamber (2), which has, for both circuits (5, 5'), connections towards the outside to a pumping means (7) and the fluid reservoirs (6, 6'), which also act as a pressure compensation receptacle.

7. Cardiovascular prostheses according to claims 1 through 6, which can be produced by means of a perfusion circuit comprised of an inner perfusion circuit (5) for exposing the blood contact side prosthesis surface to a flow along the main prosthesis axis within the chamber (2), with the prosthesis (1) being attached in the inner space by means of an adapter (3), and the prosthesis itself thus forming the inner perfusion circuit (5), as well as an outer perfusion circuit (5') joining the inner perfusion circuit (5) after having flown along the prosthesis (I) inside of chamber (2), for exposing the prosthesis (1) to an outer flow within the same chamber (2), which has, for both circuits (5, 5'), connections towards the outside to a pumping means (7) and the fluid reservoirs (6, 6'), which also act as a pressure compensation receptacle.

8. Cardiovascular prostheses according to claims 6 and 7, **characterized in that** the outer perfusion circuit (5') may be operated in a cocurrent or countercurrent flow to the inner perfusion circuit (5), or else may be operated statically.

9. Cardiovascular prostheses according to claims 6 through 8, **characterized in that** the perfusion circuits lead from one fluid reservoir (6) into another fluid reservoir (6'), where the fluid is collected that has already flown through the prosthesis.

10. Cardiovascular prostheses according to claims 6 through 8, **characterized in that** the inner and outer perfusion circuits have various fluid reservoirs or one and the same fluid reservoir (6, 6').

11. Cardiovascular prostheses according to claims 6 through 8, **characterized in that** the prosthesis is present in the fluid reservoir itself and thereby, the inner and outer perfusion circuits are connected to each other.

12. Cardiovascular prostheses according to claims 6 through 11, **characterized in that** the fluid reservoirs are comprised of expandable blood bags made of the materials PVC or EVAM.

13. Cardiovascular prostheses according to claims 6 and 7, **characterized in that** the configuration of the adapters (3, 3') for attaching the prosthesis (1) is possible as a hose coupling, a cone including a clamping device or as an expanding member.

14. Cardiovascular prostheses according to claims 6, 7 and 13, **characterized in that** the length of the prosthesis to be clamped may be varied by the constructional design of at least one terminal part with the adapter (3 or 3') of chamber (2).

15. Cardiovascular prostheses according to claims 6 and 7, **characterized in that** the chamber (2) is manufactured from a transparent material.

16. Cardiovascular prostheses according to claims 1 through 15, **characterized in that** a vascular prosthesis, a heart valve prosthesis or a stent is used as the prothesis.

17. A method of coating cardiovascular prostheses according to claims 1 through 16 with endothelial cells, **characterized in that**, after an initial subconfluent population of the blood contact side prosthesis surface, the formation of a confluent monolayer takes place by cell growth under permanent influence of defined pulsatile shearing forces increasing up to physiological values, by exposing the blood contact side prosthesis surface to a flow along the prosthesis main axis in an inner perfusion circuit, and by wetting the outer prosthesis wall in an outer perfusion circuit or in a fluid reservoir that may be flown through.

18. The method according to claim 17, **characterized in that**
a) the increasing shearing forces are generated by means of a program-controlled pumping means (7),
b) the mathematical quantity of the increasing shearing forces may be selected freely and independent of time;
c) the mathematical quantity and the final value of the shearing forces may be selected freely and time-variably in adequacy to the physiological conditions of the implantation site via a program control,
d) the mathematical quantity of the shearing forces occurring may be adjusted both by variation of the pumping performance and by variation of the cross-sectional size of the pumping tubes used or of other connection members outside of the chamber, as well as by the geometrical shape and configuration of the chamber itself.

19. The method according to claims 17 and 18, **characterized in that** in an inner perfusion circuit (5) for exposing the blood contact side prosthesis surface to a flow along the main prosthesis axis within the chamber (2), the prosthesis (1) is attached in the inner space by means of adapters (3, 3'), and the prosthesis itself thus forms the inner perfusion circuit (5), and **in that** there is an outer perfusion circuit (5') for exposing the prosthesis (1) to an outer flow within the same chamber (2), which has, for both circuits (5, 5'), connections towards the outside to a pumping means (7) and the fluid reservoirs (6, 6'), which also act as a pressure compensation receptacle.

20. The method of claims 17 through 19, **characterized in that**
a) the outer perfusion circuit (5') may be operated in a cocurrent or countercurrent flow to the inner perfusion circuit (5), or else may be operated statically,
b) the two perfusion circuits (5, 5') do not work as a closed system but lead from one fluid reservoir (6) into another fluid reservoir (6'), where the fluid is collected that has already flown through the prosthesis,
c) the inner and outer perfusion circuits have various fluid reservoirs or one and the same fluid reservoir (6, 6'),
d) the two perfusion circuits (5, 5') join each other inside of chamber (2) after having flown along the prosthesis (1), but leave the chamber (2) in separate perfusion circuits (5, 5').

21. The method according to claims 17 through 20, **characterized in that** the prosthesis is present in the fluid reservoir itself, and thereby the inner and outer perfusion circuits are connected to each other.

## Revendications

1. Prothèses cardio-vasculaires comportant une surface de cellule endothéliale pouvant être fabriquée en ce que, après une population subconfluente initiale de la surface côté contact avec le sang, se produit la formation d'une monocouche confluente par croissance des cellules sous l'action permanente de forces de cisaillement pulsatives définies croissant à des valeurs physiologiques au moyen d'un écoulement baignant la surface de prothèse côté contact avec le sang le long de l'axe principal de prothèse en un circuit de perfusion interne et un mouillage de la paroi extérieure de prothèse en un circuit de perfusion externe ou dans un réservoir de fluide pouvant être traversé par l'écoulement.

2. Prothèses cardio-vasculaires selon la revendication 1, **caractérisées en ce que** les forces de cisaillement croissantes sont générées au moyen d'un dispositif de pompage (7) piloté par programme.

3. Prothèses cardio-vasculaires selon les revendications 1 et 2, **caractérisées en ce que** la grandeur mathématique des forces de cisaillement croissantes peut être choisie variable et indépendante du temps.

4. Prothèses cardio-vasculaires selon l'une quelconque des revendications 1 à 3, **caractérisées en ce que** la grandeur mathématique et la valeur finale des forces de cisaillement peuvent être choisies, en adéquation avec les conditions physiologiques du lieu d'implantation, librement et variable dans le temps par un pilotage par programme.

5. Prothèses cardio-vasculaires selon l'une quelconque des revendications 1 à 4, **caractérisées en ce que** la grandeur mathématique des forces de cisaillement se présentant peut être réglée tant par variation de la puissance de la pompe qu'également par variation de la taille de la section transversale du tuyau de pompe utilisé ou d'autres éléments de liaison à l'extérieur de la chambre ainsi que par la forme géométrique et la réalisation de la chambre elle-même.

6. Prothèses cardio-vasculaires selon l'une quelconque des revendications 1 à 5 pouvant être fabriquée au moyen d'un circuit de perfusion se composant d'un circuit de perfusion interne (5) pour un écoulement baignant de la surface de prothèse côté contact avec le sang le long de l'axe principal de prothèse à l'intérieur de la chambre (2), la prothèse (1) étant fixée dans l'espace intérieur au moyen d'adaptateurs (3, 3') et formant ainsi elle-même le circuit de perfusion interne (5) ainsi qu'un circuit de perfusion externe (5') pour un écoulement baignant extérieur de la prothèse dans la même chambre (2) qui présente vers l'extérieur pour les deux circuits (5, 5') des liaisons avec un dispositif de pompage (7) ainsi que les réservoirs de fluide (6, 6') qui fonctionnent également en tant que récipients d'équilibrage de pression.

7. Prothèses cardio-vasculaires selon l'une quelconque des revendications 1 à 6 pouvant être fabriquée au moyen d'un circuit de perfusion se composant d'un circuit de perfusion interne (5) pour un écoulement baignant de la surface de prothèse côté contact avec le sang le long de l'axe principal de prothèse à l'intérieur de la chambre (2), la prothèse (1) étant fixée dans l'espace intérieur au moyen d'un adaptateur (3) et formant ainsi elle-même le circuit de perfusion interne (5) ainsi qu'un circuit de perfusion externe (5') s'unifiant avec le circuit de perfusion interne (5) après écoulement baignant de la prothèse (1) à l'intérieur de la chambre (2) qui présente vers l'extérieur pour les deux circuits (5, 5') des liaisons avec un dispositif de pompage (7) ainsi que les réservoirs de fluide (6, 6') qui fonctionnent également en tant que récipients d'équilibrage de pression.

8. Prothèses cardio-vasculaires selon les revendications 6 et 7, **caractérisées en ce que** le circuit de perfusion externe (5') peut être exploité en courant équivalent ou inverse par rapport au circuit de perfusion interne (5) mais aussi statiquement.

9. Prothèses cardio-vasculaires selon l'une quelconque des revendications 6 à 8, **caractérisées en ce que** les circuits de perfusion conduisent d'un réservoir de fluide (6) dans un autre réservoir de fluide (6') dans lequel est intercepté le fluide qui a déjà traversé la prothèse.

10. Prothèses cardio-vasculaires selon l'une quelconque des revendications 6 à 8, **caractérisées en ce que** les circuits de perfusion interne et externe possèdent plusieurs réservoirs de fluide ou un seul et même réservoir de fluide (6, 6').

11. Prothèses cardio-vasculaires selon l'une quelconque des revendications 6 à 8, **caractérisées en ce que** la prothèse figure dans le réservoir de fluide lui-même et qu'ainsi les circuits de perfusion interne et externe sont reliés ensemble.

12. Prothèses cardio-vasculaires selon l'une quelconque des revendications 6 à 11, **caractérisées en ce que** les réservoirs de fluide se constituent de poches à sang extensibles en matériau PVC ou EVAM.

13. Prothèses cardio-vasculaires selon les revendications 6 et 7, **caractérisées en ce que** la réalisation de l'adaptateur (3, 3') pour la fixation de la prothèse (1) est possible en tant qu'olive, que cône avec dispositif de pression ou en tant qu'élément expanseur.

14. Prothèses cardio-vasculaires selon les revendications 6, 7 et 13, **caractérisées en ce que** la longueur de la prothèse à serrer peut être modifiée par la conception constructive d'au moins une pièce de terminaison avec l'adaptateur (3 ou 3') de la chambre (2).

15. Prothèses cardio-vasculaires selon les revendications 6 et 7, **caractérisées en ce que** la chambre (2) est réalisée en un matériau transparent.

16. Prothèses cardio-vasculaires selon l'une quelconque des revendications 1 à 15, **caractérisées en ce que**, en tant que prothèse, une prothèse vasculaire, une prothèse de valvule ou un stent est employé.

17. Procédé de revêtement de prothèses cardio-vasculaires avec des cellules endothéliales selon l'une quelconque des revendications 1 à 16, **caractérisé en ce que**, après une population subconfluente initiale de la surface côté contact avec le sang, se produit la formation d'une monocouche confluente par croissance des cellules sous l'action permanente de forces de cisaillement pulsatives définies croissant à des valeurs physiologiques au moyen d'un écoulement baignant la surface de prothèse côté contact avec le sang le long de l'axe principal de prothèse en un circuit de perfusion interne et un mouillage de la paroi extérieure de prothèse en un circuit de perfusion externe ou dans un réservoir de fluide pouvant être traversé par l'écoulement.

18. Procédé selon la revendication 17, **caractérisé en ce que**
a) les forces de cisaillement croissantes sont générées au moyen d'un dispositif de pompage (7) piloté par programme,
b) la grandeur mathématique des forces de cisaillement croissantes peut être choisie variable et indépendante du temps,
c) la grandeur mathématique et la valeur finale des forces de cisaillement peuvent être choisies, en adéquation avec les conditions physiologiques du lieu d'implantation, librement et variable dans le temps par un pilotage par programme,
d) la grandeur mathématique des forces de cisaillement se présentant peut être réglée tant par variation de la puissance de la pompe qu'également par variation de la taille de la section transversale du tuyau de pompe utilisé ou d'autres éléments de liaison à l'extérieur de la chambre ainsi que par la forme géométrique et la réalisation de la chambre elle-même.

19. Procédé selon les revendications 17 et 18, **caractérisé en ce que**, en un circuit de perfusion se composant d'un circuit de perfusion interne (5), pour un écoulement baignant de l'espace intérieur de prothèse le long de l'axe principal de la prothèse à l'intérieur de la chambre (2), la prothèse (1) est fixée dans l'espace intérieur au moyen d'adaptateurs (3, 3') et forme ainsi elle-même le circuit de perfusion interne (5) et qu'un circuit de perfusion externe (5') pour un écoulement baignant extérieur de la prothèse(1) est présent dans la même chambre (2) qui présente vers l'extérieur, pour les deux circuits (5, 5'), des liaisons avec un dispositif de pompage (7) ainsi que les réservoirs de fluide (6, 6') qui fonctionnent également en tant que récipients d'équilibrage de pression.

20. Procédé selon l'une quelconque des revendications 17 à 19, **caractérisé en ce que**
a) le circuit de perfusion externe (5') peut être exploité en courant équivalent ou inverse par rapport au circuit de perfusion interne (5) mais aussi statiquement,
b) les deux circuits de perfusion (5, 5') ne fonctionnent pas en tant que système fermé mais conduisent d'un réservoir de fluide (6) dans un autre réservoir de fluide (6') dans lequel est intercepté le fluide qui a déjà traversé la prothèse,
c) les circuits de perfusion interne et externe possèdent plusieurs réservoirs de fluide ou un seul et même réservoir de fluide (6, 6'),
d) les deux circuits de perfusion (5, 5') s'unissent à l'intérieur de la chambre (2) après écoulement baignant de la prothèse (1) mais quittent la chambre (2) en des circuits de perfusion séparés (5, 5').

21. Procédé selon l'une quelconque des revendications 17 à 20, **caractérisé en ce que** la prothèse se trouve dans le réservoir de fluide lui-même et que de ce fait les circuits de perfusion interne et externe sont reliés ensemble.
